# EUROPEAN PATENT APPLICATION

(11) **EP 1 249 222 A1**
(43) Date of publication of application: **16.10.2002**
(21) Application number: 01901410.9
(22) Date of filing: 17.01.2001
(51) Int. Cl.: A61K 7/00, A61K 7/02, A61K 7/043, A61K 7/025, A61K 7/032

(54) **BRILLIANT COSMETICS**

(30) Priority: 18.01.2000 JP 2000009563
(71) Applicant: SAKURA COLOR PRODUCTS CORPORATION, Osaka-shi, Osaka 540-8508 (JP)
(72) Inventor: HIROTA, Keiko, Sakura Color Products Corp., Osaka-shi, Osaka 540-8508 (JP); YAMAMOTO, Yuki, Sakura Color Products Corp., Osaka-shi, Osaka 540-8508 (JP)
(74) Representative: Darby, David Thomas
(86) International application number: JP0100285
(87) International publication number: WO01052794

(57) **Abstract**

The cosmetics of the present invention contain scaly glass flake particles having a smooth metal surface, and the median diameter of the glass flake particles is 10 to 500 µm, and said glass flake particles are contained in 0.1 to 20 % by weight with respect to the total amount of the cosmetic composition.

## Description

### Technical field

This invention relates to cosmetics, particularly to cosmetics for makeup which realizes a strong glittering feeling and a spatial effect.

### Prior art

Heretofore, cosmetics including so called pearlescent pigments and the like in which metal oxide is coated on a surface have been provided as cosmetics for makeup including lip sticks, nail polishes, and eye shadows, and the like (Japanese Unexamined Patent Publications S62-187770, H6-116507, H9-132514, H8-302236, and 2000-186012).

However, with these conventional cosmetics, those with a strong glitter and a spatial effect have not always been provided so far.

The object of the present invention is to provide cosmetics, particularly, cosmetics for makeup which are capable of manifesting a stronger glittering feeling and further, a stronger spatial effect.

### Disclosure of the invention

As a result of intensive studies to achieve the above object, the inventors have found that the cosmetics with a strong glittering effect and further, a strong spatial effect can be provided if the cosmetics comprise scaly glittering particles which have a smooth surface of a metal (with alloys included), that is, a smooth metal surface. Further, the inventors have found that in the present invention, the cosmetics are preferable which include glass flake particles whose surface is coated with a metal, inorganic particles whose surface is coated with a metal, metal foil powders, and metal coated resin film powders and that the cosmetics which include multi layer film powders are also preferable.

In glittering cosmetics including scaly glittering particles with such a metal surface provided, an incident light to the metal surface reflects on the said smooth metal surface (total refection is preferable) and as a result of it, the cosmetic coated films having both a strong glittering feeling and a spatial effect in which glitters are interspersed on the surface of skins, hairs, nails, and the like that have not been provided in the past can be obtained.

On the other hand, as an example which is similar but more different from glittering particles whose particle surface is coated with a metal, the particles whose surfaces are coated with metal oxide as pearlescent pigments mentioned above are cited. However, in the case of these glittering particles, metal oxide layers are transparent and they have a strong tendency to be colored due to the property of metal oxide. Further, the incident light reflects not on the surface of metal oxide, but the light incidents inside the metal oxide layer and is refracted, and the interference of the light inside the metal oxide layer occurs, thereby making it difficult to obtain a strong glittering feeling and a spatial effect.

On the other hand, in the case of cosmetics which include scaly glittering particles having a smooth metallic surface, particularly, scaly glass flake particles whose particle surface is coated with a metal, as mentioned above, as a result that the incident light directly reflects on a smooth metal surface provided on a smooth surface of scaly glass flake particles, a strong glittering feeling and a spatial effect can be provided by the reflection of the light on this metal surface, and in the case of the cosmetics which include these, particularly in the case of the cosmetics for makeup, a strong glittering feeling and a spatial effect with a glitter can appear on nails and the like on which these cosmetics are coated.

### Brief explanation of drawings

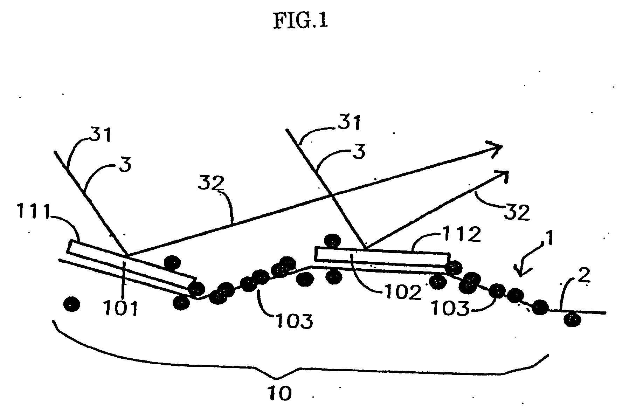

Fig.1 is a schematic sectional view of the cosmetic coated films illustrated as a model when the cosmetics of the present invention are coated on a nail surface.

### Best mode for carrying out the invention

### (Glittering particles)

As glittering particles used in the present invention, it is important that they are scaly glittering particles (glittering fragment) having a smooth metal surface. In particular, it is preferable to be scaly glittering particles having a smooth surface on which the incident light is reflected totally. Although the said metals are not specifically limited, metallic simple substance or metallic alloy whose light reflectance is not less than 0.5 and examples include silver (reflectance; 0.94), aluminum (reflectance; 0.83), gold (reflectance; 0.80), nickel (reflectance; 0.63) and the like.

The said glitter is achieved when scaly glittering particles have smooth metal surface, however, in the case of the glittering particles with a small particle diameter of less than 10µm, a glittering feeling is not enough and it is difficult to obtain a cosmetic coated film having a strong glittering feeling and a spatial effect which have not been achieved in the past in which a glitter (glittering fragment) is interspersed even though the cosmetic coated film can be made to have a metallic luster. Therefore, the size of the particle diameter affects this glittering feeling and the median diameter of the said scaly glittering particles is at least 10µm, at least 25µm, preferably not less than 30µm, and more preferably, with a big particle diameter up to 500µm.

For information, the "median diameter" of the present invention represents the value of D₅₀ (µm) measured by laser diffraction using a median diameter measuring device under the trade name of "Microtrac HRA 9320-X100" manufactured by Leeda & Northlup Co., Ltd.

According to the present invention, when the cosmetics are coated on a human nail surface and the like and a cosmetic coated film is formed, in the said coated film, the bigger the value of the median diameter of D₅₀ of the glittering particles is, and the smaller the maximum value of the surface roughness (JIS B 0601), Rₘₐₓ, the stronger glittering feeling appears.

Consequently, when the minimum value of a glittering feeling to which both of the property values relate is related as a ratio of smoothness to the said median diameter (smoothness (µm) / median diameter (µm)), particularly when its ratio is not greater than 0.011, a glittering feeling is strongly realized. For information, "smoothness" defined in the present invention is obtained by measuring the maximum value of each surface roughness of glittering particles which are present per unit area of the surface of a cosmetic coated film in a dried state by using the electronic microscope under the trade name of "ERA-8000" manufactured by ELIONIX, thereafter calculating the average value (µm) of these maximum value Rₘₐₓ.

For information, in the embodiment of the present invention, glittering particles can be used for cosmetics including facial cosmetics, makeup cosmetics hair cosmetic materials, and the like, particularly they can preferably be used for nail polishes, pedicures, nail polishes, mascaras, eyeliners, eye shadows, lipsticks, cheeks, foundations, or the like without limited to aqueous or oily, regardless of being in solid or in liquid.

In liquid, it is preferable that the cosmetics have pseudo-plasticity fluidity (thixotropic property). With this property, settling can be prevented and the aggregated gathering of scaly glittering particles can be prevented even when scaly glittering particles with a big particle diameter are included, and when the said cosmetics are coated, a cosmetic coated film can be obtained with the said glitter interspersed which have not been realized in the past.

When colorants are included in the cosmetics, the glittering particles of the present invention is preferably the ones in which the coating ratio of the colorants is not greater than 80%. Here, the "coating ratio of the colorants" shows the coating ratio of the colorants which include coloring pigments and the like covering the surface of a particle and is represented by a coating area of the colorant to the total superficial area of a particle surface. In the present invention, in a surface of cosmetic coated film, as for plurality of glittering particles per unit area, by making digital images taken by a polarization microscope "OPTIPHOT" manufactured by Nikon Corporation and a digital camera "HC-300Z" manufactured by Fuji Photo Film Co., Ltd binary with an image processing software "Image Plus" manufactured by Horie-MS, the superficial area of the said particles and the coating area of the colorants are measured, and the coating ratio is calculated taking the average value. This "coating ratio of the colorants" is the property value that can be adjusted by the size of glittering particles and of colorants, amount to be contained, and dispersibility in the cosmetics of each particle as well as viscosity of cosmetics or the like.

In the present invention, as base particles of glittering particles, as mentioned above, glass flake particles, inorganic particles, metal foil powders, metal coated resin film powders, multi layer film powders and the like can be cited. In other words, in the present invention, scaly glittering particles having a smooth metal surface have the structure in which the said base particles are coated with a metal. Here, although "coating" not only includes the total of the particle surface, but also includes a part of the particle surface, glittering particles whose total particle surface is coated with a metal are preferable. This similarly applies not only to glass flake particles, but also to particles with other metal coating structure hereinafter.

To cite one example as glass flake particles whose particle surface is coated with a metal, glass flake particles coated with a metal by an electronless plating method can be used. Examples include glass flake particles coated with silver under the trade names of "METASHINE REFSX-2015PS", "METASHINE REFSX-2025PS", "METASHINE REFSX-2040PS" manufactured by TOYO ALUMINIUM K.K. and "METASHINE RCFSX-5480PS", "METASHINE RCFSX-5230PS", "METASHINE RCFSX-5150PS", and "METASHINE RCFSX-5090PS" manufactured by Nippon Sheet Glass Co., Ltd.

In addition, glass flake particles whose surface is coated with a metal of flaky glasses by a sputtering method can also be used. Examples include glittering particles coated with silver under the trade names of "CHRYSTAL COLOR-GF2125", "CHRYSTAL COLOR-GF2125-M", "CHRYSTAL COLOR-GF2140", and "CHRYSTAL COLOR-GF2140-M" manufactured by TOYO ALUMINIUM K.K. Further, the examples of the particles whose surface is coated with nickel- chrome-molybdenum alloy include the products manufactured by TOYO ALUMINIUM K.K. under the trade names of "CHRYSTAL COLOR-GF2525", "CHRYSTAL COLOR-GF2525 M", "CHRYSTAL COLOR-GF2540", and "CHRYSTAL COLOR-GF2540 M". Other examples include "CHRYSTAL COLOR-GF250"manufactured by TOYO ALUMINIUM K.K. coated with brass, "CHRYSTAL COLOR-GF1345" manufactured by TOYO ALUMINIUM K.K. coated with silver alloy, and "CHRYSTAL COLOR-GF1445" manufactured by TOYO ALUMINIUM K.K. coated with titanium.

The median diameter of the glass flake particles of the present invention whose particle surface is covered with a metal is preferably not greater than 500µm, and particularly 10 to 500µm. When the median diameter exceeds 500µm, slippage occurs at the time of coating and the finish is not preferable. For information, when the cosmetics of the present invention are filled in a ball-point pen type cosmetic container and is coated on a surface of a human skin or nail surface with the rotation of a ball at the tip, the median diameter of glittering particles is preferably set not greater than 100µm from the viewpoint of fluidity.

The said glass flake particles in the present invention are preferably contained in 0.1 to 20 % by weight with respect to the total amount of the cosmetic composition. When the glass flake particles are contained in less than 0.1% by weight, a glittering feeling and a spatial effect are not satisfactory. On the other hand, when the said glass flake particles exceed 20% by weight with respect to the total amount of the cosmetic composition, the solid contents become too much and the finish of the cosmetic coated film is not beautiful and the usability deteriorates, too. The optimum content of the glass flake particles is 0.5 to 10 % by weight.

For information, the said glass flake particles in the present invention can be used alone or in combinations of two or more of them. Further, the said glass flake particles can be used by mixing with glass flake particles with the particle surface of metal oxide provided, with the metal coated inorganic particles mentioned hereinafter, and with glittering pigments including aluminum pigments or pearlescent pigments, or the like.

### (Inorganic particles)

The inorganic particles used in the present invention are inorganic particles having a smooth metal surface (excluding glass flake particles). The examples include inorganic particles coated with a metal by metal deposition or the like. To be specific, mica-like iron oxide (III) whose surface is coated with aluminum -manganese can be used. Examples include the trade names "Paliocrom Copper L 3000" and "Paliocrom Copper L 3001" manufactured by BASF Ltd.

The median diameter of inorganic particles having the said smooth metal surface is not greater than 500 µm, preferably 10 to 500 µm as the median diameter of the said glass flake particles. When the median diameter of the metal coated inorganic particles exceeds 500 µm, the slippage occurs at the time of coating and the finishing state is not preferable.

In addition, even in the case of aluminum pigments, as stated above, when the scaly aluminum powders having a median diameter of at least 10 µm and having a smooth surface, preferably, the scaly aluminum powders having the ratio of the said smoothness is not greater than 0.011 are used, the twinkling glitter which the said particles inherently have can be realized in a coated film. Examples of such aluminum powders capable of providing these property values include the trade names "WXM U 75 C" (median diameter: 13 µm), "WXM 5422" (median diameter: 18 µm), "WXM 1440" (median diameter: 30 µm), "WXM 1415" (median diameter: 50 µm) manufactured by TOYO ALUMINIUM K.K. Particularly, in the case of aluminum powders, trade names "WXM 1440" (median diameter: 30 µm) and "WXM 1415" (median diameter: 50 µm) manufactured by TOYO ALUMINIUM K.K. can preferably be used.

Particularly, in the case of aluminum powders, the trade names "WXM 1440" (median diameter: 30 µm) and "WXM 1415" (median diameter: 50 µm) manufactured by the above manufacturer whose particle median diameter is not less than 30 µm, that is, over 25 µm can preferably be used.

The said inorganic particles are, as the said glass flake particles, preferably contained in 0.1 to 20 % by weight with respect to the total amount of the cosmetic composition. When the said inorganic particles is contained in less than 0.1 % by weight with respect to the total amount of the cosmetic composition, the glittering feeling is not enough, and when the content of the inorganic particles exceeds 20 % by weight with respect to the total amount of the cosmetic composition, the solid contents become too much, the finish is not beautiful and the usability deteriorates. The optimum amount of the said inorganic particles to be contained is 0.1 to 10 % by weight.

For information, the said inorganic particles having a smooth metal surface can be used alone or in combinations of two or more of them. In addition, the said inorganic particles can be used not only with glass flake particles whose particle surface is covered with a metal but also with glittering pigments including aluminum pigments and pearlescent pigments in mixture.

### (Metal foil powders)

The metal foil powders used in the present invention are defined as general terms for metallic powders in which the metal foils including aluminum foils are grounded. Therefore, in the present invention, metal foil powders with such definition can be used. However, in the case of glittering cosmetics which contain the aluminum foil powders, whose foil surface is processed by mirror surface treatment, and in particular, in the case where the flakes are block-type, when used for make up, it is preferable in that a stronger glittering feeling and a spatial effect can be provided. In addition, in the case of aluminum foil powders colored by pigments or dyes as well as whose surface is processed with mirror surface treatment, such a make up can be obtained with a strong glittering feeling and a spatial effect having the color tone that has not been realized in the past, according to the hue colored by pigments and dyes.

To cite an example, Silver, part number H 25, DG.Gold, the same part number, LG.Gold, the same part number, Green, the same part number, Blue, the same part number, Red, the same part number, Maroon, the same part number, and Black, the same part number under the trade name of "Diamond piece H type" in which a foil surface is colored as well as conducted mirror surface treatment manufactured by DAIYA KOGYO CO., LTD, Silver, part number H 55, DG.Gold, the same part number, LG.Gold, the same part number, Green, the same part number, Blue, the same part number, Red, the same part number, Maroon, the same part number, and Black, the same part number can be cited.

Further, Silver, part number #500, Gold, the same part number, Silver, part number #325, R. Gold, B. Gold, Red, Blue, Green, and Violet of "LG series" manufactured by Oike industrial Co., Ltd can be cited.

Although the granularity of this metal foil powders is not specifically limited, the granularity of 500 mesh to 50 mesh is preferable. When the granularity exceeds 500 mesh, the glittering feeling is inferior since flake particles are too small. The granularity with less than 50 mesh can still be used, however, it is preferable that the granularity is not less than 50 mesh to be contained in cosmetics.

The metal foil powder of the present invention is preferably contained in 0.01 to 20.0 % by weight with respect to the total amount of the cosmetic composition. When the said metal foil powders are contained in less than 0.01 % by weight, a glittering feeling and a spatial effect are not enough. When the said metal foil powders exceed 20.0 % by weight, the solid contents become too much, the finish is not beautiful, and the usability deteriorates. The optimal amount to be contained is 0.05 to 10.0 % by weight. In addition, the said metal foil powders in the present invention can be used with the said metal coated inorganic particles, glass flake particles whose particle surface is coated with a metal, and with glittering pigments such as aluminum pigments, pearlescent pigments or the like in mixture.

### (Metal coated resin film powders)

The metal coated resin film powders used in the present invention are composed of resin film powders coated with metals including aluminum by deposition, and the like. For example, resin film powders of polyethylene terephthalate (PET), with aluminum deposited thereon or colored resin film powders can be cited. In addition, by using a press die of hologram, resin film powders (aluminum deposited PET emboss hologram) in which aluminum is deposited on polyethylene terephthalate (PET) with invisible fine grooves embossed can be used. This PET embossed hologram glitters in rainbow colors, realizing a glittering feeling by a prism effect.

To be specific, examples including the trade names "DIAMOND PIECE (regular type) series Silver, part number 55, DG Gold, , the same part number, LG Gold, the same part number, and Green, part number 501, Blue, the same part number, Red, the same part number, Maroon, the same part number, and Black, the same part number manufactured by DAIYAKOGYO CO., LTD can be cited. Also, part numbers HG-5EP and HG-S20 of "DAIYA HOLOGRAM" series manufactured by DAIYA KOGYO CO., LTD can be cited.

The granularity of this metal coated resin film powders is not specifically limited, but the granularity of 140 mesh to 50 mesh is preferable. When the granularity exceeds 140 mesh, flake particles become too small and the glittering feeling is inferior.

The metal coated resin film powders are preferably contained in 0.01 to 20.0 % by weight with respect to the total amount of the cosmetic composition. When the said metal coated resin film powders is contained in less than 0.01 % by weight with respect to the total amount of the cosmetic composition, a glittering feeling and a spatial effect are not enough. When the content of the metal coated resin film powders exceed 20.0 % by weight with respect to the total amount of the cosmetic composition, the solid contents become too much, the finish is not beautiful, and the usability deteriorates. The optimal amount of the metal coated resin film powders to be contained is 0.05 to 10.0 % by weight. In addition, the said metal coated resin film powders can be used with the said metal foil powders, the said metal coated inorganic particles, glass flake particles on whose particle surface metal or metal oxide is coated and with glittering pigments including aluminum pigments, pearlescent pigments, or the like in mixture.

### (Multi layer film powders)

The multi layer film powders in the present invention refer to the fine powders of the multi layer films and the synthetic resin film powders in which colors change depending on the viewing angles due to the color development of multi layer with the refraction of light. This "multi layer film powders" include multi layer film powders colored with high transparency.

The multi layer film powders are not specifically limited so long as they are color materials capable of developing color of a glittery feeling by having a mode of a multi layer film. Such multi layer film powders include "Crystal color" series and "Rainbow flake" series manufactured by DAIYAKOGYO CO., LTD, and the like.

Examples of the said "crystal color" series include part numbers "X-5", "X-20", "X-40", "X-701-30", "X-701-10" and in each of the part numbers, there are color tones including opal, topaz, blue topaz, emerald, coral, sapphire, diamond, aqua marine, peridot, and blue moon. In other words, series of "crystal color X-20" include trade names "crystal color X-20 OPAL", "crystal color X-20 TOPAZ", "crystal color X-20 BLUE TOPAZ", "crystal color X-20 EMERALD", "crystal color X-20 CORAL", "crystal color X-20 SAPHIRE", "crystal color X-20 DIAMOND", "crystal color X-20 AQUA MARINE", "crystal color X-20 PERIDOT", and "crystal color X-20 BLUE MOON". Therefore, there are trade names which correspond to the above including "crystal color X-5" series, "crystal color X-40" series, "crystal color X-701-30" series, and "crystal color X-701-10" series.

In addition, "Rainbow flake" series include part numbers "No55", "No501", "No510", "No530", "No550", "No580", "NoR-05", "NoR-15", and "No501-30" and each of the part numbers, there are color tones including crystal, lemon yellow, deep yellow, apricot, Nile green, green, pink, sky blue, royal blue, lavender, red, and monarch green. In other words, series of "Rainbow flake No 55" include trade names "Rainbow flake No 55 crystal", "Rainbow flake No 55 lemon yellow", "Rainbow flake No 55 deep yellow", "Rainbow flake No 55 apricot", "Rainbow flake No 55 Nile green", "Rainbow flake No 55 green", "Rainbow flake No 55 pink", "Rainbow flake No 55 sky blue", "Rainbow flake No 55 royal blue", "Rainbow flake No 55 lavender", "Rainbow flake No 55 red", and "Rainbow flake No 55 monarch green". Therefore, there are trade names which correspond to the above including series of "Rainbow flake No 501", series of "Rainbow flake No 510", series of "Rainbow flake No 530", series of " Rainbow flake No 550", series of "Rainbow flake No 580", series of "Rainbow flake NoR-05", series of "Rainbow flake NoR-15", and series of "Rainbow flake NoNo501-30".

The multi layer film powders are preferably contained in 0.01 to 20 % by weight with respect to the total amount of the cosmetic composition. When the glass flake particles are contained in less than 0.01% by weight with respect to the total amount of the cosmetic composition, a glittering feeling and a spatial effect are not satisfactory. On the other hand, when the multi layer film powders exceed 20% by weight with respect to the total amount of the cosmetic composition, the solid contents become too much, the finish is not beautiful and the usability deteriorates. The optimum content of the multi layer film powders is 0.05 to 10 % by weight. In addition, the said multi layer film powders can be used with the said metal coated resin film powders, the said metal foil powders, the said metal coated inorganic particles, glass flake particles whose particle surface is coated with a metal, and with glittering pigments including aluminum pigments, pearlescent pigments, or the like in mixture. For information, the granularity of the multi layer film powders is preferably 40 mesh to 200 mesh.

In the cosmetics of the present invention, various components used as ordinary cosmetics, particularly cosmetics for make up can appropriately be selected and used in so far as they do not inhibit the effect of the present invention. These components include colorants (coloring matter including pigments, dyes, or the like), oily components including solid oils, semi solid oils, liquid oils, or the like, further, organic solvents, moisturizing agents, resins, thickening agents, waxes and oils, plasticizers, ultra violet ray absorbing agents, anti oxidants, antiseptics, disinfectants, surfactants, perfume materials, water, powdery materials, pH modifiers, water soluble polymers, beauty components, and the like.

Examples of oily components include high aliphatic alcohols, high aliphatic acids, ester oils, paraffin oils, waxes, and the like. Examples of organic solvents include alcohols such as ethyl alcohols, propylene glycols, sorbitols, glucoses, and the like, as well as ethyl acetate, acetone, and toluene. Examples of moisturizing agents include muco polysaccharids, collagens, PCA salts, lactate, and the like. And as for surfactants, various kinds of surfactants can be used including nonionic types, cationic types, anionic types, and ampholyte types. Further, examples of thickening agents include pullulan, xanthan gum, welan gum, rhamsan gum, succinoglucan, dextran, tragacannth gum, guar gum, tara gum, locust bean gum, ghatti gum, arabinogalactan gum, gum arabic, quince seed gum, pectin, starch, psyllium seed gum, pectin, carageenan, alginic acid, agar, gelatin, casein, albumin, polyvinyl pyrrolidone, ethyl cellulose, carboxy methyl cellulose, carboxy vinyl polymer, N- vinyl acetamide type resins. As for these thickening agents, those capable of providing pseudo plasticity fluidity (thixotropic property) are included.

As for colorants, various kinds of dyes can be used which are usually compounded in cosmetics including C.I Acid red 27 (Red No.2), C.I Acid red 51 (Red No.3), C.I Acid red 18 (Red No.102), C.I Acid red 28 (Red No.104), C.I Acid red 94 (Red No.105), C.I Acid red 52 (Red No.106), C.I Acid yellow 23 (Yellow No.4), C.I Acid yellow 3 (Yellow No.5), C.I Food green 3 (Green No.3), C.I Food blue 2 (Blue No.1), C.I Acid blue 74 (Blue No.2), and the like as well as various kinds of pigments (coloring pigments and / or body filler pigments), including inorganic pigments such as titanium oxide, graphite, colcothar, carbon black, ultramarine blue pigment, kaolin, aluminum oxide, magnesium carbonate, calcium carbonate, and organic pigments such as C.I. pigment black 1 (Black No401) C.I. pigment green 7, C.I. pigment blue 15 (Blue No4), C.I. pigment red 112, C.I pigment violet 19, Watching red, C.I. pigment 57-1(Red No201), C.I. pigment 57 (Red No202), and various kinds of other pigments which are usually compounded such as organic tar pigments, lake pigments with organic coloring mater, and the like.

The production method of the cosmetics of the present invention is obtained by mixing each component as mentioned above. Upon mixture, a known method on each kind of cosmetics can be adopted. The mode of the cosmetics of the present invention can be various including stick types, powder types, liquid types, emulsion types, gelling types, cake types, cream types, pencil types, and the like. Stick types are preferable when coating on cheeks or lips, for example, which can be obtained by solidification with waxes contained. Powder types are preferable when coating on cheeks as rouge compositions, for example, which can be obtained by involving inorganic fillers such as kaolin, aluminum oxide, magnesium carbonate, calcium carbonate, and the like. The mode of the above cosmetics is the one that can be obtained by involving a known carrier component. In addition, the cosmetics can be used as the ones of transfer types which are transferred to the skin, or the like.

Further, the cosmetics of the present invention can be applied to ball point pen type applicators (ball point pen type containers) with a ball retained in a pen tip, and the cosmetics filled in the container, which are coated on skins, nail surfaces, or the like with the rotation of a ball.

In this case, in the reservoir of the container, liquid cosmetics or semi liquid cosmetics such as moisturizers and the like can be filled. However, by involving the said thickening agents which provide pseudo plasticity fluidity (thixotropic property), it is also possible to apply to the ball point pen type applicators (ball point pen type containers) which have never been in the past in which the cosmetics are coated on skins, nail surfaces, and the like with the sudden lowering of the viscosity due to a shear force acting on the cosmetics although they are gel type (non-flow or hard to flow) cosmetics when they are contained in the said applicators.

The cosmetics of the present invention can also be applied not only to the ball pen tip, but also to the pen type applicators (pen type containers) comprising fiber bundles, or the like. The cosmetics of the present invention can not only applied to the provision structure of the direct liquid type, but also be applied to the inner lead type in which the cosmetics are impregnated in the inner lead of fiber bundles or the like or the bulb type applicators (pen type containers) in which the cosmetics are provided to the pen lead via a bulb. However, in the case of pen type applicators (pen type containers) in which scaly glittering particles with a smooth metal surface and the large particle diameter of at least 10 µm, particularly not less than 30µm are contained in the cosmetics, it is preferable to apply to the containers in which cosmetics with pseudo plasticity fluidity (thixotropic property) provided, particularly, to the ball pen type applicators (ball point pen type containers). No doubt, the present application can be applied to the liquid or semi liquid type cosmetics with Newtonian fluidity property.

When the mode of the present invention is aqueous, it is preferable to use synthetic resin emulsion with acrylic types, styrene-acrylic types, vinyl acetate types, and the like.

In the present invention, since the dimension of glittering particles is large, it is sometimes difficult to fix the base particles of glittering particles and the like firmly on the coated films, the glittering particles are easily peeled due to rubbing or the like after coating, and sometimes the generation of a strong glittering feeling and a spatial effect on cosmetic coated films are hard to be realized with the low durability of cosmetic coated film having a glittering feeling and a spatial effect. From the above, when cosmetics include scaly glittering particles having a large particle diameter and a smooth metal surface, particularly, the said glass flake particles, it is desirable to include binder components which fix the said glittering particles on a coated films.

### (Forming method of cosmetic coated films and coated films)

Although the forming method of the cosmetic coated films of the present invention is not specifically limited, it is desirable that the scaly glittering particles having the median diameter of at least 10µ m and having a smooth metal surface are scattered and interspersed in the cosmetic coated films. In particular, in utilizing cosmetics which include colorants(coloring pigments), such method is preferable that the scaly glittering particles are distributed with the ratio of not greater than 80% to the coated films as a whole and the said colorant particles are distributed between the said glittering particles.

Fig. 1 is a schematic sectional view of a cosmetic coated film illustrated as a model when the cosmetics of the present invention are coated with the above method. As shown in Fig.1, in the present embodiment, the said glittering particles 101 are distributed on the surface of nail 2 (coated surface) to nail surface 10 as a whole with the distribution ratio of not greater than 80 %, and coated film 1 can be formed in which colorant particles 103 are distributed between glittering particles 101 and glittering particles 102. Detailed explanation according to the present embodiment goes that, in this coated film 1, the said glittering particles 101 and 102 are, from the microscopic view point, deposed on the surface of nail 2 (coated surface) maintaining the smooth surface to the uneven surface which composes the said surface and further, between glittering particles 101 having this smooth surface and glittering particles 102 having a smooth surface, coloring particles 103 are distributed as particle groups of the colorant. In the embodiment of the present invention, two glittering particles 101 and 102 show coated films, however, they are naturally plural of glittering particles and it is important that it is a coated film between which the said glittering particles, and the colorants are distributed and the particle groups of the colorants are deposed. By adopting such a forming method of a coated film, in glittering particles, with the presence of an uneven surface which composes the surface of nail 2 (coated surface), plural of smooth surfaces with various angles are interspersed on the surface of a coated film and since these glittering particles are preferably scaly glittering particles in which the coating ratio of the colorants covering the surface of the said particles is not greater than 80 % in a state of a dried coated film, the incidence of a light to the particles of the colorants covering the surface of particles 101 and 102 are none or very little, and the reflected light to each of the smooth surfaces of glittering particles are not disturbed and incidence 31 and reflection 32 of light 3 are obtained according to the smooth surface of glittering particles. Further, as shown in Fig 1, for example, since incidence 31 and reflection 32 of light 3 with different angles to smooth surfaces 111 and 112 occur, a twinkling glitter is realized since the viewing angle of the coated films subtly changes.
Further, in the present embodiment, since the glittering particles having the said property value are distributed with the distributing ratio of preferably not greater than 80 % to the coated film surface as a whole and since the coated film is in such a way that between the glittering particles coloring particles are distributed, the color development is maintained in good balance, providing a twinkling glitter and a color development each other synergistically, thereby capable of providing colored glittering feeling to a coated film. For information, when the said glittering particles are distributed with a distributing ratio of over 80% to the coated film as a whole on the nail surface (coated surface), even though the colorants are present between the plurality of glittering particles, a glittering feeling of glittering particles overcomes the color development of coloring particles on the surface of a coated film, which inhibits color development of the said colorants. Moreover, a visual glittering feeling of glittering particles appearing on a surface of a coated film is not decided solely by an area occupied by glittering particles on a surface of a coated film, but rather, by contrast, a glittering feeling deteriorates since the colored surface of glittering particles are composed together with the lowering of color development of colorant particles when glittering particles occupy most of the coated film surface. In other words, since a glittering feeling of glittering particles is a visual perception given off synergistically in relation to distribution of colorants which are present among glittering particles, it is preferable that this glittering feeling is distributed with the distribution ratio of not greater than 80 % to a whole coated surface in relation to distribution of colorants. For information, the "distributing ratio" of glittering particles in the present invention is a numerical value (%) obtained by making the digital image taken by a polarization microscope "OPTIPHOT" manufactured by Nikon Corporation and a digital camera "HC-300Z" manufactured by Fuji Photo Film Co., Ltd binary by an image processing software "Image Plus" manufactured by Horie-MS, measuring the area of glittering particles to the total area of a coated film, thereby calculating the ratio. Here, it is further preferable that the scaly glittering particles of the present invention are made to be particle sin which the coating ratio of colorants which cover the surface of the said particles is not greater than 40 % in a state of a dried coated film. Further, it is the most suitable that the distribution ratio of these scaly glittering particle sis between the range of 20 to 45 % with respect to the total area of a coated film.

### EXAMPLES

Each cosmetic material was obtained by mixing each of the components with the composition and compounding amount (wt%) as shown in Tables 1 to 3 with a known production method of each cosmetic material. To be specific, Example 1 and Comparative Examples 1 to 2 are for lipsticks, Example 2 and Comparative Examples 3 to 4 are for nail polishes, and Example 3 and Comparative Examples 5 to 6 are for eye shadows.

**Table 1**

| (Lip sticks) | | | | |
|---|---|---|---|---|
| | | Example 1 | Comparative Example 1 | Comparative Example 2 |
| | | wt% | wt% | wt% |
| Ozokerite | | 5.00 | 5.00 | 5.00 |
| Ceresin | | 5.00 | 5.00 | 5.00 |
| Solid paraffin | | 10.00 | 10.00 | 10.00 |
| Glyceryl tri-2-ethyl hexanoic acid ester | | 20.00 | 20.00 | 20.00 |
| Diisostrearyl malate | | 40.25 | 40.25 | 40.25 |
| Octyl dodecyl myristate | | 10.00 | 10.00 | 10.00 |
| Tocopherol acetate | | 0.20 | 0.20 | 0.20 |
| Butyl parahydroxy benzoate | | 0.05 | 0.05 | 0.05 |
| Glass flake pigments | | 7.00 | | |
| Aluminum flake powder pigments | | | 7.00 | |
| Pearlescent pigments | | | | 7.00 |
| Red No.201 | | 1.50 | 1.50 | 1.50 |
| Red No.202 | | 1.00 | 1.00 | 1.00 |
| Total | | 100.00 | 100.00 | 100.00 |
| Evaluation | Glittering feeling | ○ | Δ | Δ |
| | Spatial effect | ○ | Δ | Δ |

**Table 2**

| (Nail polishes) | | | | |
|---|---|---|---|---|
| | | Example 2 | Comparative Example 3 | Comparative Example 4 |
| | | wt% | wt% | wt% |
| Cellulose nitrate | | 15.00 | 15.00 | 15.00 |
| Sucrose benzoate | | 5.00 | 5.00 | 5.00 |
| Toluene sulfonic amide resin | | 5.00 | 5.00 | 5.00 |
| Alkyd resin | | 5.00 | 5.00 | 5.00 |
| Acetyl tributyl citrate | | 4.00 | 4.00 | 4.00 |
| Ethyl acetate | | 15.00 | 15.00 | 15.00 |
| Butyl acetate | | 43.50 | 43.50 | 43.50 |
| Isopropyl alcohol | | 5.00 | 5.00 | 5.00 |
| Organic bentonite gelling agent | | 1.00 | 1.00 | 1.00 |
| Glass flake pigments | | 1.00 | | |
| Aluminum flake powder pigments | | | 1.00 | |
| Pearlescent pigments | | | | 1.00 |
| Red No.202 | | 0.50 | 0.50 | 0.50 |
| Total | | 100.00 | 100.00 | 100.00 |
| Evaluation | Glittering feeling | ○ | Δ | Δ |
| | Spatial effect | ○ | Δ | Δ |

**Table 3**

| (Eye shadow) | | | | |
|---|---|---|---|---|
| | | Example 3 | Comparative Example 5 | Comparative Example 6 |
| | | wt% | wt% | wt% |
| Talc | | 43.00 | 43.00 | 43.00 |
| Sericite | | 40.80 | 40.80 | 40.80 |
| Zinc stearate | | 5.00 | 5.00 | 5.00 |
| Liquid paraffin | | 3.00 | 3.00 | 3.00 |
| Glass flake pigments | | 8.00 | | |
| Aluminum flake powder pigments | | | 8.00 | |
| Pearlescent pigments | | | | 8.00 |
| Red No. 202 | | 0.20 | 0.20 | 0.20 |
| Total | | 100.00 | 100.00 | 100.00 |
| Evaluation | Glittering feeling | ○ | Δ | Δ |
| | Spatial effect | ○ | Δ | Δ |

In the above tables 1 to 3, a trade name "METASHINE REFSX-2025 PS" was used for the glass flake whose particle surface is coated with a metal (In the table, it is shown as "glass flake pigment") manufactured by TOYO ALUMINIUM K.K. with a median diameter of about 25 µm was used. A trade name "WXM0630" manufactured by TOYO ALUMINIUM K.K with an average diameter of 5µm was used for aluminum flake pigments. A trade name "IRIODIN 302" manufactured by Merck Japan Co., Ltd with an average particle diameter of about 5 to 20 µm was used as pearlescent pigments coated with metallic oxide.

### (Evaluation Test)

Next, with these cosmetics, a make up was applied depending on each application, followed by the following tests the evaluation results of which were put down therewith in Tables 1 to 3.

### (Glittering feeling and spatial effect)

With these cosmetics, a make up was applied depending on each application, followed by the evaluation on a glittering feeling and a spatial effect of each of the cosmetics. A glittering feeling was evaluated by visual observation of a make up state and the rating criteria were: ○ for an extremely strong glittering feeling; Δ for a strong glittering feeling; and × for little or no glittering feeling. A spatial effect was also evaluated by visual observation of a make up state and the rating criteria were: ○ for an extremely strong spatial effect; Δ for a strong spatial effect; and × for little or no spatial effect.

When the lip stick of Example 1 was used, a make up with a strong glittering feeling and a spatial effect can be provided on lips.

And when the nail polish of Example 2 was used, a make up with a strong glittering feeling and a spatial effect can be provided on nails. Further, when the eye shadow of Example 3 was used, a strong glittering feeling and a spatial effect can be provided on eyes.

In the present examples, since they are glittering cosmetics comprising glass flake particles whose particle surface is coated with a metal, compared with the conventional cosmetics using pearlescent pigments and aluminum flake particles (median diameter is less than 10µm, non-smooth surface) , a special kind of a make up with a stronger glittering feeling and a spatial effect can be provided which has never been in the past.

Further, as shown in Table 4, in the case of glittering cosmetics (nail polishes) of Examples 4 to 7 which comprise metal coated inorganic particles, metal coated resin film powders, metal foil powders, and multi-layer film powders respectively, a strong glittering feeling and a spatial effect can also be provided. For information, the glittering cosmetics comprising the above mentioned glass flake particles have a stronger glittering feeling than the glittering cosmetics which comprise metal coated inorganic particles, metal coated resin film powders, metal foil powders, and multi-layer film powders respectively and they are the most suitable when used particularly for nail polishes including hand nail polishes and pedicures as well as and eye shadows.

**Table 4**

| (Nail polishes) | | | | | |
|---|---|---|---|---|---|
| | | Example 4 | Example 5 | Example 6 | Example 7 |
| | | wt% | wt% | wt% | wt% |
| Cellulose nitrate | | 15.00 | 15.00 | 15.00 | 15.00 |
| Sucrose benzoate | | 5.00 | 5.00 | 5.00 | 5.00 |
| Toluene sulfonic amide resin | | 5.00 | 5.00 | 5.00 | 5.00 |
| Alkyd | | 5.00 | 5.00 | 5.00 | 5.00 |
| Acetyl tributyl citrate | | 4.00 | 4.00 | 4.00 | 4.00 |
| Ethyl acetate | | 15.00 | 15.00 | 15.00 | 15.00 |
| Butyl acetate | | 43.50 | 43.50 | 43.50 | 43.50 |
| Isopropyl alcohol | | 5.00 | 5.00 | 5.00 | 5.00 |
| Organic bentonite gelling agent | | 1.00 | 1.00 | 1.00 | 1.00 |
| Metal coated inorganic particles | | 1.00 | | | |
| Metal coated resin film powders | | | 1.00 | | |
| Metal foil powders | | | | 1.00 | |
| Multi layer film powders | | | | | 1.00 |
| Red No.202 | | 0.50 | 0.50 | 0.50 | 0.50 |
| Total | | 100.00 | 100.00 | 100.00 | 100.00 |
| Evaluation | Glittering feeling | ○ | ○ | ○ | ○ |
| | Spatial effect | ○ | ○ | ○ | ○ |

For information, in the above Table 4, as metal coated inorganic particles, mica like iron oxide(III) whose surface is coated with aluminum-manganese was used. And as metal coated resin film powders, Silver, part number 55 under the trade name of "DIAMOND PIECE (regular type)" manufactured by DIA KOGYO CO., LTD was used. Further, as metal foil powders, Silver, part number H25 under the trade name of "DIAMOND PIECE H type" manufactured by DIA KOGYO CO., LTD was used. As multi layer film powders, "CRYSTAL COLOR X-20 OPAL" manufactured by DIA KOGYO CO., LTD was used.

### Industrial Applicability

The cosmetics of the present invention are useful as cosmetics for various purposes including facial cosmetic materials, makeup cosmetic materials, hair cosmetic materials, and the like. In particular, they are preferably used for nail polishes, pedicures, nail polishers, mascaras, eye liners, eye shadows, lip sticks, foundations, or the like.

## Claims

1. Glittering cosmetics comprising scaly glittering particles having a smooth metal surface.

2. Glittering cosmetics as set forth in claim 1, wherein said glittering particles have a median diameter of at least 10µm.

3. Glittering cosmetics as set forth in claim 2, wherein the ratio of smoothness on a particle surface to the median diameter of said glittering particles is not greater than 0.011.

4. Glittering cosmetics as set forth in claim 1, having pseudo-plasticity fluidity (thixotropic property).

5. Glittering cosmetics as set forth in claim 1, wherein base particles of said glittering particles are glass flake particles.

6. Glittering cosmetics as set forth in claim 1, wherein said metal is metallic simple substance or alloy of any one of silver, gold, nickel, or aluminum.

7. Glittering cosmetics as set forth in claim 5, wherein said metal is coated on a surface of glass flake particles.

8. Glittering cosmetics as set forth in claim 5, wherein a median diameter of said glass flake particles is 10 to 500µm.

9. Glittering cosmetics as set forth in claim 5, wherein said glass flake particles are contained in 0.1 to 20 % by weight with respect to the total amount of the cosmetic composition.

10. Glittering cosmetics as set forth in claim 1, wherein base particles of said glittering particles are inorganic particles.

11. Glittering cosmetics as set forth in claim 10, wherein a median diameter of said inorganic particles is 10 to 500µm.

12. Glittering cosmetics as set forth in claim 1, wherein base particles of said glittering particles are metal foil powders.

13. Glittering cosmetics as set forth in claim 12, wherein said metal foil powders are aluminum foil powders with mirror surface treatment.

14. Glittering cosmetics as set forth in claim 12, wherein said metal foil powders are contained in 0.01 to 20.0 % by weight with respect to the total amount of the cosmetic composition.

15. Glittering cosmetics as set forth in claim 1, wherein base particles of said glittering particles are resin film powders.

16. Glittering cosmetics as set forth in claim 15, wherein said resin film powders are polyethylene terephthalate film powders with aluminum deposited.

17. Glittering cosmetics as set forth in claim 15, wherein said resin film powders are contained in 0.01 to 20.0 % by weight with respect to the total amount of the cosmetic composition.

18. Glittering cosmetics as set forth in claim 1, wherein base particles of said glittering particles are multi layer film powders.

19. Glittering cosmetics as set forth in claim 18, wherein said multi layer film powders are contained in 0.01 to 20.0 % by weight with respect to the total amount of the cosmetic composition.

20. Glittering cosmetics as set forth in claim 1, comprising a binder component for fixing said scaly glittering particles to human skins, hairs, or nails.

21. Glittering cosmetics as set forth in claim 20, comprising synthetic resin emulsion as said binder component.

22. A lipstick comprising glittering cosmetics as set forth in claim 1.

23. A nail polish comprising glittering cosmetics as set forth in claim 1.

24. An eye shadow comprising glittering cosmetics as set forth in claim 1.

25. A method of forming coated films in glittering cosmetics comprising scaly glittering particles having a median diameter of at least 10µm and a smooth metal surface, wherein colorant particles are distributed among the glittering particles.

26. A method of forming coated films in glittering cosmetics comprising scaly glittering particles having a median diameter of at least 10µm and a smooth metal surface, the ratio of smoothness on the particle surface to said median diameter is not greater than 0.011, the coating ratio of the colorants covering the surface of said particles is not greater than 80% in a state of a dried coated film, and the coating ratio of colorants covering said particle surface and these scaly glittering particles are distributed with a distributing ratio of not greater than 80% to the coated film surface as a whole, wherein the colorant particles are distributed among said glittering particles.

27. A coated film of cosmetics provided with a glittering feeling and a spatial effect.

28. A coated film as set forth in claim 27, wherein scaly glittering particles with a smooth metal surface are sprinkled and interspersed.

29. A coated film wherein scaly glittering particles have a median diameter of at least 10µm and a smooth metal surface, wherein colorant particles are distributed among glittering particles.

30. A coated film for cosmetics in glittering cosmetics comprising scaly glittering particles having a median diameter of at least 10µm and a smooth metal surface, the ratio of smoothness on the particle surface to said median diameter being not greater than 0.011, the coating ratio of the colorants covering the surface of said particles is not greater than 80% in a state of a dried coated film and the coating ratio of colorants covering said particle surface and these scaly glittering particles being distributed with a distributing ratio of not greater than 80% to the coated film surface as a whole, wherein said colorant particles are distributed among said glittering particles.

31. A ball-point pen type applicator wherein cosmetics comprising scaly glittering particles with a smooth metal surface are filled in a reservoir.

32. A ball-point pen type applicator as set forth the in claim 31, wherein scaly glittering particles are glittering particles selected from a group of glass flake particles with a smooth metal surface, aluminum particles with a smooth particle surface, and inorganic particles coated with a metal.

33. A ball-point pen type applicator as set forth in claim 31, wherein scaly glittering particles have a median diameter of at least 10 µm.

34. A ball-point pen type applicator as set forth in claim 33, wherein the ratio of smooth ness of said scaly glittering particles to said median diameter on a particle surface is not greater than 0.011 and the coating ratio of the colorants covering the surface of said particles is not greater than 80 % in a state of a dried coated film.
